# EUROPEAN PATENT SPECIFICATION

(11) **EP 3 003 207 B1**
(45) Date of publication and mention of the grant of the patent: **25.09.2019**
(21) Application number: 14807523.7
(22) Date of filing: 03.06.2014
(51) Int. Cl.: A61F 5/56, B29C 51/02, B29C 51/10, A61C 7/08

(54) **DENTAL APPLIANCE SYSTEM AND METHOD OF MANUFACTURE**
DENTALVORRICHTUNGSSYSTEM UND VERFAHREN ZUR HERSTELLUNG
SYSTÈME D'APPAREIL DENTAIRE ET PROCÉDÉ DE FABRICATION

(30) Priority: 04.06.2013 US 201361830994 P; 04.06.2013 US 201361830755 P
(43) Date of publication of application: 13.04.2016
(73) Proprietor: Frantz, Donald, Katy, Texas 7794 (US)
(72) Inventor: FRANTZ, Joseph, Austin, Texas 78703 (US); KINCHEN, Dane, Hammond, Louisiana 70401 (US)
(74) Representative: AWA Sweden AB
(86) International application number: PCT/US2014/040763
(87) International publication number: WO 2014/197516

(56) References cited:
- WO-A1-2009/155223
- DE-A1-102008 061 325
- DE-A1-102010 036 107
- DE-U1-202013 001 392
- GB-A- 2 192 359
- US-A- 5 597 525
- US-A- 5 829 980
- US-A1- 2002 144 691
- US-A1- 2009 155 736
- US-A1- 2011 039 223
- US-A1- 2012 197 002
- US-B1- 6 584 978

## Description

This application claims priority to US Provisional Patent Application Nos. 61/830,994 and 61/830,755 both filed on June 4, 2013 entitled DENTAL APPLIANCE SYSTEM AND METHOD OF MANUFACTURE and ELASTICLY ADJUSTABLE MANDIBULAR APPLIANCE AND METHOD OF TREATING SLEEP APNEA AND SNORING AND PREVENTING OR REDUCING THE RISK OF SIDE EFFECTS THEREOF.

### Technical Field

The present application relates generally to dental appliance manufacture and methods therefor.

### Background

It is well documented in the literature that an oral appliance that opens the bite and moves the mandible forward will greatly reduce sleep apnea and snoring. It is also documented that these appliances are capable of producing considerable discomfort to patients, unwanted movement of their teeth, and/or tempromandibular joint (TMJ) pain as well as other problems.

A variety of trial oral appliances are available for preventing snoring and sleep apnea. Of these, all are removable, and most advance the mandible. Most dental sleep appliances have been made of bulky "boil & bite" material. These appliances have not been effective and have not had patient acceptance or compliance. These uncomfortable devices have driven patients away from wanting to be treated by a custom oral appliance.

Also, several removable, oral snoring/apnea appliances are adjustable, pulling the jaw forward in different, set percentages of their maximum movement. However, no existing appliance is ideally adjustable, both in amount of forward movement and vertical opening. Instead, temporary or permanent adjustments to appliances are made by either placing spacers, turning screws, or by grinding away plastic or other material. These modifications change the amount of advancement; however, a danger in locking the patient's jaw in one ridged spot is causing TMJ pain. Once modifications are made, however, they are permanent until further modified by the doctor. In summary, appliances exist in which the amount of advancement may be changed, but the changes result in a new fixed position of the mandible many times creating pain and discomfort. Other appliances do not offer easily changed vertical or caudal displacement of the mandible to increase the effectiveness of the appliance.

Another problem with plastic oral appliances, including those custom built in a laboratory based on a mold of a patient's teeth, is durability. Appliances that are vacuum molded are typically made from a plastic that was previously extruded and cut to create discs for the vacuum forming process. What is needed is an adjustable oral snoring/sleep apnea appliance which is effective, which has high patient acceptance, and which will not cause temporomandibular joint problems, unwanted tooth movement or soreness. Another need is an appliance that is resilient enough to withstand nightly use without breakage and yet provide comfort.

The appliance of the present invention is to greatly reduce, or eliminate, sleep apnea and snoring, while alleviating temporomandibular joint problems, unwanted tooth movement and soreness, with complete adjustability of the appliance both in the amount of forward movement of the lower jaw, and in the amount of vertical bite opening. Another object is a mandibular advancement appliance with high patient acceptance, comfort, and resilience for treatment success.

DE102008061325 discloses a method for producing a multilayer dental splint in a thermoforming device. DE102008061325 discloses a first thermoforming sheet and at least one second thermoforming sheet and optionally at least a third thermoforming sheet are thermoformed by deep drawing on a jaw model, wherein the first thermoforming sheet and the second thermoforming sheet or the third thermoforming sheet comprises a hard layer of a first plastic material and at least one thermoforming sheet of a soft layer a second plastic material and wherein the thermoforming sheets are connected to one another such that a layer composite with a hard outer layer; on each of the two outer sides and with a soft between the hard layers embedded inner layer is obtained.

### Summary

One aspect of the present invention is a method for reparing a dental treatment appliance comprising a tray molded to cover a patient's teeth, the method comprising: injection overmolding a thermoplastic material with a thermoplastic elastomer material to form a chemically bonded material; and thermoforming the injection overmolded chemically bonded material to form the tray.

An another aspect of the invention is a dental appliance comprising: a tray molded to cover a patient's teeth, the tray formed by injection overmolding a thermoplastic material with a thermoplastic elastomer material to form a chemically bonded material and thermoforming the overmolded layered composite polymeric material, the tray including the thermoplastic material of a first durometer configured for an exterior portion of the tray and the thermoplastic material of a second durometer configured for an interior portion of the tray.

The foregoing is a summary and thus contains, by necessity, simplifications, generalizations and omissions of detail; consequently, those skilled in the art will appreciate that the summary is illustrative only and is NOT intended to be in any way limiting, the scope of the present invention being defined by the appended claims. Other aspects, features, and advantages of the devices and/or processes and/or other subject described herein will become apparent in the text set forth herein.

### Brief Description of the Drawings

A better understanding of the subject matter of the application can be obtained when the following detailed description of the disclosed embodiments is considered in conjunction with the following drawings, in which:
Figure 1 is a flow diagrams of a method in accordance with an embodiment of the subject matter of the present application.
Figure 2 illustrates a vacuum forming device for thermal forming plastic to a dental mold in accordance with an embodiment of the subject matter of the present application.
Figure 3 illustrates a dental appliance in accordance with an embodiment of the subject matter of the present application.

The use of the same symbols in different drawings typically indicates similar or identical items.

### Detailed Description of the Drawings

In the description that follows, the subject matter of the application will be described with reference to acts and symbolic representations of operations that are performed by one or more machines, and/or computers, unless indicated otherwise.

In each of the embodiments presented, a dental appliance is formed by injection molding polymeric material; and thermoforming the injection molded chemically bonded material to form a dental appliance. Importantly, instead of using extruded material in a vacuum forming instrument, an injection molded material is used. It has been discovered that the molecules of an extruded plastic that is vacuum formed for dental appliances become weaker through the thermoprocessing, resulting in a weaker, brittle product. Embodiments herein are directed to different dental appliance techniques achieved by injection molding plastic for dental appliances instead of using extruded plastics. The injection molding process allows not only for a stronger material, but avoids the requirement of glues or lamination processes by enabling overmolding.

Material used for the discs currently being used in the dental industry to make devices such as splints, TMJ pain appliances, bleaching trays, and sleep appliances are manufactured from extruded material. The extrusion process is one that forces all of the molecules in the material to be aligned in one direction. The current manufacture of the material is producing 4x8 foot sheets of plastic from which the discs are cut. This creates a disc that is strong in one direction, but creates weakness in other directions, thereby giving inconsistent results. According to one embodiment, an injection molding process creates discs directly instead of cutting discs from a sheet of material already created by an extrusion process.

As a result of using an injection molding process, a tougher material results in a thinner but stronger dental device than extruded material. After thermo-forming the injection molded disc, a more effective dental device results that provides increased tongue space. Further, a harder, tougher, more dense injection molded disc, even though thinner which will last longer without distortion or breakage allowing tooth movement and dislodgement of the appliance.

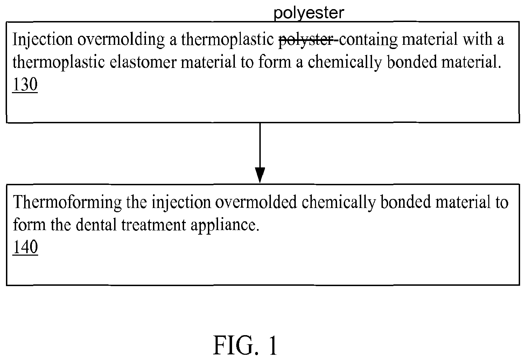

Referring now to Figure 1, another embodiment includes method for preparing an overmolded dental treatment appliance is shown. Block 130 provides for injection overmolding a thermoplastic polyster material with a thermoplastic elastomer material to form a chemically bonded material. Block 140 provides for thermoforming the injection overmolded chemically bonded material to form the dental treatment appliance. The thermoforming can be vacuum forming such that the thermoplastic elastomer material side of the chemically bonded material adheres to a mold of the patient's teeth.

In one embodiment, the injection molding via an overmolding process uses a copolyester and a thermoplastic elastomer (TPE). For example, the overmolding can use a polyethylene terephtalate (PTE) or a glycol-modified polyethylene terephthalate (PTEG) as with a thermoplastic elastomer (TPE). To perform the overmolding, the injection molding can include overmolding via a single injection molding cycle using a two-shot process.

As a result of the overmolding, a dental appliance results that includes a tray molded to cover a patient's teeth, the tray made of a thermoplastic material of a first durometer configured for an exterior portion of the tray, and thermoplastic material of a second durometer configured for an interior portion of the tray.

For example, an overmolding of a polyethylene terephtalate (PTE) with a thermoplastic elastomer (TPE) will result in a softer durometer and a harder durometer tray such that an interior portion of the tray is softer than than the exterior portion of the tray.

In one embodiment, the dental appliance can include a second tray using one or more of the processes described herein. For example, the second tray can be an amorphous thermoplastic and the first tray can be formed by thermally molding an overmolded layered composite polymeric material or vice versa.

In one embodiment, one or more arcuate members can be added to the trays for releasably attaching an elastic band to enable the dental appliance to treating sleep apnea.

The material is, preferably a material approved by the U.S. Food and Drug Administration for use in the oral cavity such as PETG 6763 and GLS TPE 3060-1.

In one embodiment, the dental appliance includes a elastomer polymeric material that is flexible, lightweight, translucent and adapts to the contours of the teeth to permit a self-retentive frictional contact between the device and the associated upper and lower posterior teeth. The flexible material chemically bonded to a firm amorphous material via injection molding to provide resiliency while also providing retention where necessary. The dual-sided appliance will provide better usage by the patient over the homogenously hard plastic material of prior appliances. In one embodiment, the elastomer material is of a thickness of between 30 and 200 shoreA durometers.

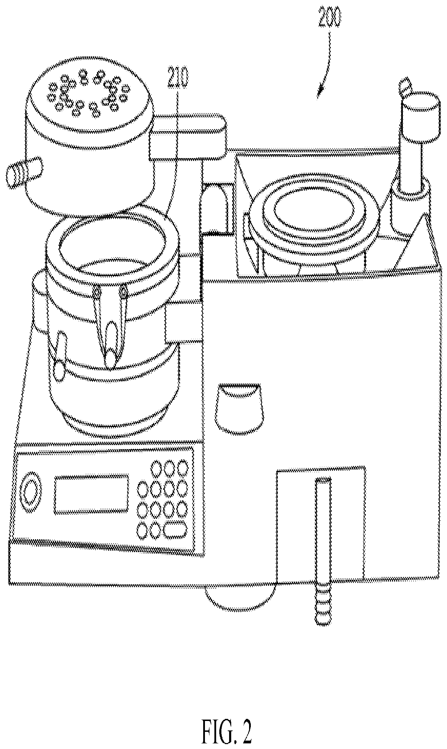

As will be appreciated by those of skill in the art, the thermoforming can be by vacuum forming as shown in Figure 2. In one embodiment, the thermoforming can be via an alternative to vacuum forming in which exact shape of the teeth is not necessary.

For example, the elastomer material, being a softer material, can preferably be thermal formed over a mold of a patient's teeth. The amorphous, harder, material can preferably be thermal formed on the outside of the mold such that the softer material touches the patient's teeth when the dental appliance is inserted.

For example, an "over-shot" of the elastomer material can be during a 60 second injection molding cycle.

By overmolding, the two materials become one injection overmolded chemically bonded material to form the dental treatment appliance. Thus, the overmolding creates a stronger bond than lamination or any uses of glues. One overmolding process is the two-shot injection molding, which involves injecting one material into a mold to form the initial part or section of a part. Once formed, a second material is injected into the mold to form the remainder of the part. Two-shot molding requires that the two materials be compatible (chemically similar), or no bonding occurs. In embodiments herein, the materials are harder and softer versions of thermoplastics that enable a harder amorphous material to be infused with a softer elastomer material.

The two shot, or dual shot process can be accomplished by injecting one material (usually hard material) in a mold and immediately injection shooting a second material (soft) while the first material is still hot. The material for the hard portion of the disk is Eastman copolyester PETG 6763 or copolyester Tritan TX-1000, for example, the first shot injection molding. The material for the second shot can be a GLS/Polyone TPE 3060-1.

The following examples illustrate to one of ordinary skill in the art of thermoplastics how to manufacture the material to enable bonding without the use of glues.
Material: Tritan + PETG copolyester
Example 1.
   Drying Temperature 88°C (190°F)
   Drying Time 4-6 hrs
   Processing Melt Temperature 260-282°C (500-540°F)
   Mold Temperature 38-66°C (100-150°F)
   Material: GLS/PolyOne Versaflex 3060-1
Example 2.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 3.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 4.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 5.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 6.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 7.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 8.
   Rear Barrel Temperature (177 - 216 °C) (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 9.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 10.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 11.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125 rpm
Example 12.
   Rear Barrel Temperature 177 - 216 °C (351 - 421 °F)
   Middle Barrel Temperature 182 - 221 °C (360 - 430 °F)
   Front Barrel Temperature 193 - 232 °C (379 - 450 °F)
   Nozzle Temperature 204 - 243 °C (399 - 469 °F)
   Mold Temperature 21.1 - 32.2 °C (70.0 - 90.0 °F)
   Back Pressure 0.517 - 1.21 MPa (75.0 - 175 psi)
   Screw Speed 75.0 - 125

Referring now to Figure 2, in a machine typically seen in a dentist's office, such as a thermo-plastic machine 200. The dentist can place discs of prepared injection molded plastic 210 in the machine so that upon heating and applying a vacuum, the sheets are pulled down over the model 420 of the patent's upper and lower teeth, one at a time. The machine can operate on the upper and lower teeth models separately, as will be appreciated by those skilled in the art.

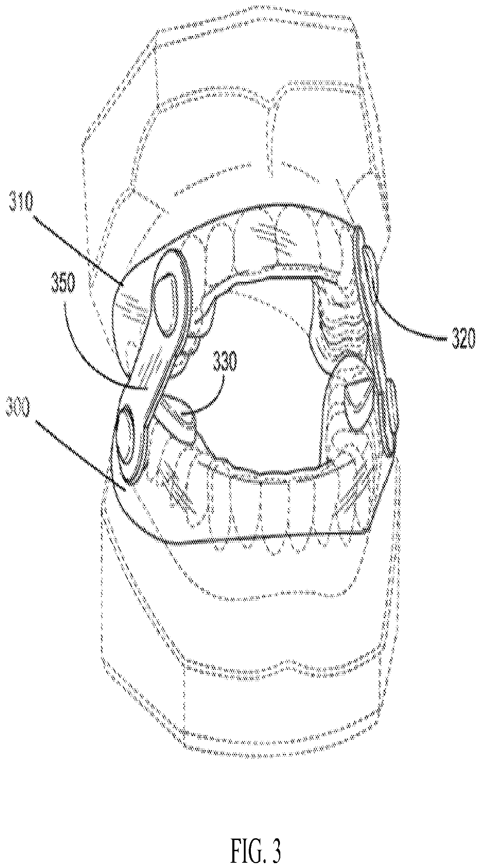

Referring now to Figure 3, an embodiment is directed to dental appliance. The dental appliance can include dental tray 300 and second dental tray 310, such that each is a tray molded to cover a patient's teeth, the tray formed by thermoforming an overmolded layered composite polymeric material,. Each of tray 300 and 310 can have a thermoplastic material of a first durometer configured for an exterior portion of the tray. For example the teeth of the patient can be directly in contact with the thermoplastic elastomer material such that an exact fit is not necessary. It has been discovered that the thermoplastic elastomer material can create a softer dental appliance when injection molding via an overmolding of a polyethylene terephtalate (PTE) with a thermoplastic elastomer (TPE). The TPE, being a softer material allows for a more comfortable fit on the patient's teeth. Each of tray 300 and 310 can further include attachments 320 and/or 330. Attachments 320 enable one or more elastic bands to releasably attach each of tray 300 and 310. Attachments 330 enable a vertical displacement between trays 300 and 310 when inserted into a patient's mouth.

While the subject matter of the application has been shown and described with reference to particular embodiments thereof, it will be understood by those skilled in the art that the scope of the present invention is defined by the appended claims.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation *is* explicitly recited, those skilled in the art will recognize that such recitation should typically be interpreted to mean *at least* the recited number (e.g., the bare recitation of "two recitations," without other modifiers, typically means *at least* two recitations, or *two or more* recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.).

## Claims

1. A method for preparing a dental treatment appliance comprising a tray molded to cover a patient's teeth, the method
comprising:
injection overmolding a thermoplastic material with a thermoplastic elastomer material to form a chemically bonded material; and
thermoforming the injection overmolded chemically bonded material to form the tray.

2. The method of claim 1, wherein the thermoforming the injection overmolded chemically bonded material to form the tray includes:
vacuum forming the chemically bonded material onto a model of a patient's teeth such that the thermoplastic elastomer material side of the chemically bonded material adheres to a mold of the patient's teeth.

3. The method of claim 1, wherein the injection overmolding a thermoplastic material with a thermoplastic elastomer material to form a chemically bonded material includes:
injection molding via an overmolding process using a copolyester and a thermoplastic elastomer (TPE).

4. The method of claim 1, wherein the injection overmolding a thermoplastic material with a thermoplastic elastomer material to form a chemically bonded material includes:
injection molding via an overmolding of a polyethylene terephtalate (PTE) with a thermoplastic elastomer (TPE).

5. The method of claim 4, wherein the injection molding via an overmolding of a polyethylene terephtalate (PTE) with a thermoplastic elastomer (TPE) includes:
overmolding via a single injection molding cycle using a two-shot process.

6. The method of claim 4, wherein the injection molding via an overmolding of a polyethylene terephtalate (PTE) with a thermoplastic elastomer (TPE) includes:
overmolding of the PTE wherein the PTE is a glycol-modified polyethylene terephthalate (PTEG).

7. A dental appliance comprising:
a tray molded to cover a patient's teeth, the tray formed by injection overmolding a thermoplastic material with a thermoplastic elastomer material to form a chemically bonded material and thermoforming the overmolded layered composite polymeric material, the tray including:
the thermoplastic material of a first durometer configured for an exterior portion of the tray; and
the thermoplastic elastomer material of a second durometer configured for an interior portion of the tray.

8. The dental appliance of claim 7 wherein the overmolded layered composite polymeric material is formed by injection molding via an overmolding of a polyethylene terephtalate (PTE) with a thermoplastic elastomer (TPE).

9. The dental appliance of claim 7 wherein the overmolded layered composite polymeric material is formed by injection molding via an overmolding of a glycol-modified Polyethylene Terephtalate (PTEG) with a thermoplastic elastomer (TPE).

10. The dental appliance of claim 7 wherein the first durometer and the second durometer are different and between 10 and 100 ShoreA.

11. The dental appliance of claim 7 wherein the dental appliance further comprises:
a second tray, wherein the second tray is formed by thermally molding an overmolded layered composite polymeric material.

12. The dental appliance of claim 7 wherein the thermal molding is a vacuum forming process using a model of the patient's teeth.

13. The dental appliance of claim 7 wherein the tray includes:
one or more arcuate members for releasably attaching an elastic band.

14. The dental appliance of claim 13 further comprising:
a second dental tray, the second dental tray including one or more attachments enabling the elastic band to releasably attach to the second tray.

15. The dental appliance of claim 13 wherein the tray and the second tray are attached via one or more elastic bands to enable the dental appliance to be removably insertable in the mouth of the patient for treating sleep apnea.

## Patentansprüche

1. Verfahren für die Herstellung einer Dentalbehandlungsvorrichtung umfassend eine Schale, die zum Überdecken der Zähne eines Patienten geformt ist, wobei das Verfahren Folgendes umfasst:
Injektionsumspritzen eines thermoplastischen Materials mit einem thermoplastischen elastomeren Material, um ein chemisch gebundenes Material zu bilden; und
Thermoformen des injektionsumspritzten, chemisch gebundenen Materials, um die Schale zu bilden.

2. Verfahren nach Anspruch 1, wobei das Thermoformen des injektionsumspritzten, chemisch gebundenen Materials zum Bilden der Schale Folgendes umfasst:
Vakuumformen des chemisch gebundenen Materials auf ein Modell der Zähne eines Patienten, derart, dass die thermoplastische elastomere Materialseite des chemisch gebundenen Materials an einer Form der Zähne des Patienten anhaftet.

3. Verfahren nach Anspruch 1, wobei das Injektionsumspritzen eines thermoplastischen Materials mit einem thermoplastischen elatomeren Material zum Bilden eines chemisch gebundenen Materials Folgendes umfasst:
Spritzgießen durch ein Umspritzungsverfahren unter Anwendung eines Copolyesters und eines thermoplastischen Elastomers (TPE).

4. Verfahren nach Anspruch 1, wobei das Injektionsumspritzen eines thermoplastischen Materials mit einem thermoplastischen elastomeren Material zum Bilden eines chemisch gebundenen Materials Folgendes umfasst:
Spritzgießen durch Umspritzen eines Polyethylenterephthalats (PTE) mit einem thermoplastischen Elastomer (TPE).

5. Verfahren nach Anspruch 4, wobei das Spritzgießen durch Umspritzen eines Polyethylenterepththalats (PTE) mit einem thermoplastischen Elastomer (TPE) Folgendes umfasst:
Umspritzen durch einen einzigen Spritzgießzyklus unter Anwendung eines Zweischussverfahrens.

6. Verfahren nach Anspruch 4, wobei das Spritzgießen durch Umspritzen eines Polyethylenterepththalats (PTE) mit einem thermoplastischen Elastomer (TPE) Folgendes umfasst:
Umspritzen des PTE, wobei das PTE ein glycolmodifiziertes Polyethylenterephthalat (PTEG) ist

7. Dentalvorrichtung umfassend:
eine Schale, die zum Überdecken der Zähne eines Patienten geformt ist, wobei die Schale, die durch Injektionsumspritzen eines thermoplastischen Materials mit einem thermoplastischen elastomeren Material, um ein chemisch gebundenes Material zu bilden, und Thermoformen des umspritzten, schichtförmigen polymeren Verbundstoffmaterials geformt ist, Folgendes umfasst:
das thermoplastische Material eines ersten Durometers, der für einen externen Teil der Schale konfiguriert ist; und
das thermoplastische elastomere Material eines zweiten Durometers, der für einen internen Teil der Schale konfiguriert ist.

8. Dentalvorrichtung nach Anspruch 7, wobei das umspritzte, schichtförmige, polymere Verbundstoffmaterial durch Spritzgießen durch Umspritzen eines Polyethylenterephthalats (PTE) mit einem thermoplastischen Elastomer (TPE) gebildet wird.

9. Dentalvorrichtung nach Anspruch 7, wobei das umspritzte, schichtförmige, polymere Verbundstoffmaterial durch Spritzgießen durch Umspritzen eines glycolmodifizierten Polyethylenterephthalats (PTEG) mit einem thermoplastischen Elastomer (TPE) gebildet wird.

10. Dentalvorrichtung nach Anspruch 7, wobei der erste Durometer und der zweite Durometer verschieden sind und 10 bis 100 Shore A aufweisen.

11. Dentalvorrichtung nach Anspruch 7, wobei die Dentalvorrichtung ferner Folgendes umfasst:
eine zweite Schale, wobei die zweite Schale durch thermisches Formen eines umspritzten schichtförmigen polymeren Verbundstoffmaterials gebildet wird.

12. Dentalvorrichtung nach Anspruch 7, wobei das thermische Formen ein Vakuumformverfahren unter Anwendung eines Modells der Zähne des Patienten ist.

13. Dentalvorrichtung nach Anspruch 7, wobei die Schale Folgendes umfasst:
ein oder mehrere bogenförmige Elemente zum freisetzbaren Befestigen eines elastischen Bands.

14. Dentalvorrichtung nach Anspruch 13, ferner Folgendes umfassend:
eine zweite dentale Schale, wobei die zweite dentale Schale einen oder mehrere Ansätze umfasst, die ermöglichen, das elastische Band freisetzbar an der zweiten Schale zu befestigen

15. Dentalvorrichtung nach Anspruch 13, wobei die Schale und die zweite Schale durch ein oder mehrere elastische Bänder befestigt werden, um zu ermöglichen, dass die dentale Vorrichtung entfernbar in den Mund des Patienten zum Behandeln von Schlafapnoe einführbar ist.

## Revendications

1. Procédé de préparation d'un appareil de traitement dentaire comprenant un plateau moulé afin de recouvrir la dent d'un patient, le procédé comprenant :
le surmoulage par injection d'un matériau thermoplastique avec un matériau élastomère thermoplastique afin de former un matériau chimiquement lié ; et
le thermoformage du matériau chimiquement lié surmoulé par injection afin de former le plateau.

2. Procédé selon la revendication 1, dans lequel le thermoformage dans le matériau chimiquement lié surmoulé par injection afin de former le plateau comprend :
la formation sous vide du matériau chimiquement lié sur un modèle d'une dent d'un patient de sorte que le côté du matériau élastomère thermoplastique du matériau chimiquement lié à terre à un moule de la dent d'un patient.

3. Procédé selon la revendication 1, dans lequel le surmoulage par injection d'un matériau thermoplastique avec un matériau élastomère thermoplastique afin de former un matériau chimiquement lié comprend :
le moulage par injection par l'intermédiaire d'un processus de surmoulage en utilisant un copolyester et un élastomère thermoplastique (TPE).

4. Procédé selon la revendication 1, dans lequel le surmoulage par injection d'un matériau thermoplastique avec un matériau élastomère thermoplastique afin de former un matériau chimiquement lié comprend :
le moulage par injection par l'intermédiaire d'un surmoulage d'un téréphtalate de polyéthylène (PTE) avec un élastomère thermoplastique (TPE).

5. Procédé selon la revendication 4, dans lequel le moulage par injection par l'intermédiaire d'un surmoulage d'un téréphtalate de polyéthylène (PTE) avec un élastomère thermoplastique (TPE) comprend :
le surmoulage par l'intermédiaire d'un cycle de moulage par injection unique au moyen d'un procédé en deux étapes.

6. Procédé selon la revendication 4, dans lequel le moulage par injection par l'intermédiaire d'un surmoulage d'un téréphtalate de polyéthylène (PTE) avec un élastomère thermoplastique (TPE) comprend :
le surmoulage du PT, dans lequel le PT est un téréphtalate de polyéthylène à glycol modifié (PTEG).

7. Appareil dentaire comprenant :
un plateau moulé pour recouvrir la dent d'un patient, le plateau étend formé par surmoulage par injection d'un matériau thermoplastique avec un matériau élastomère thermoplastique afin de former un matériau chimiquement lié et le thermoformage du matériau polymère composite en couches surmoulé, le plateau comprenant :
le matériau thermoplastique d'un premier duromètre conçu pour une partie extérieure du plateau ;et
le matériau élastomère thermoplastique d'un second duromètre conçu pour une partie intérieure du plateau.

8. Appareil dentaire selon la revendication 7, dans lequel le matériau polymère composite en couches surmoulé est formé par moulage par injection par l'intermédiaire d'un surmoulage d'un téréphtalate de polyéthylène (PTE) avec un élastomère thermoplastique (TPE).

9. Appareil dentaire selon la revendication 7, dans lequel le matériau polymère composite en couches surmoulé est formé par moulage par injection par l'intermédiaire d'un surmoulage d'un téréphtalate de polyéthylène à glycol modifié (PTEG) avec un élastomère thermoplastique (TPE).

10. Appareil dentaire selon la revendication 7, dans lequel le premier duromètre et le second duromètre sont différents et sont compris entre 10 et 100 ShoreA.

11. Appareil dentaire selon la revendication 7, dans lequel l'appareil dentaire comprend en outre :
un second plateau, dans lequel le second plateau est formé par moulage thermique d'un matériau polymère composite en couches surmoulé.

12. Appareil dentaire selon la revendication 7, dans lequel le moulage thermique est un processus de formation solide utilisant un modèle de la dent d'un patient.

13. Appareil dentaire selon la revendication 7, dans lequel le plateau comprend au moins un élément arqué permettant de fixer de manière amovible une bande élastique.

14. Appareil dentaire selon la revendication 13, comprenant en outre :
un second plateau dentaire, le second plateau dentaire comprenant au moins un dispositif de fixation permettant à la bande élastique de se fixer de manière amovible au second plateau.

15. Appareil dentaire selon la revendication 13, dans lequel le plateau et le second plateau fixé par l'intermédiaire d'au moins une bande élastique afin de permettre à l'appareil dentaire d'être inséré de manière amovible dans la bouche du patient pour le traitement de l'apnée du sommeil.
